# EUROPEAN PATENT SPECIFICATION

(11) **EP 1 867 733 B1**
(45) Date of publication and mention of the grant of the patent: **30.12.2009**
(21) Application number: 07011875.7
(22) Date of filing: 18.06.2007
(51) Int. Cl.: C12Q 1/68, B01J 19/00, B01L 3/00

(54) **Biological material preparation chip system and method for DNA extraction from biological materials**
Chipsystem zur Herstellung von biologischem Material und Verfahren zur Gewinnung von DNA aus biologischen Materialien
Système de puce pour la préparation de matériaux biologiques et procédé pour l'extraction d'ADN de matériaux biologiques

(30) Priority: 16.06.2006 JP 2006166802
(43) Date of publication of application: 19.12.2007
(73) Proprietor: HITACHI SOFTWARE ENGINEERING CO., LTD., Shinagawa-ku Tokyo 140-0002 (JP)
(72) Inventor: Sasaki, Yasuhiko, c/o Hitachi, Ltd. Intellectual Property Group, Chiyoda-ku Tokyo 100-8220 (JP); Inaba, Toru, c/o Hitachi, Ltd., Intellectual Property Group, Chiyoda-ku Tokyo 100-8220 (JP); Kishida, Hiroshi, c/o Hitachi Software Engineering Co., Ltd., Shinagawa-ku Tokyo 140-0002 (JP); Uchida, Masaomi, c/o Hitachi Software Engineering Co., Ltd., Shinagawa-ku Tokyo 140-0002 (JP)
(74) Representative: Beetz & Partner

(56) References cited:
- WO-A-99/09042
- WO-A-2005/073691
- US-A1- 2006 068 491
- JOON-HO KIM ET AL: "A disposable DNA sample preparation microfluidic chip for nucleic acid probe assay" PROCEEDINGS OF THE IEEE 15TH. ANNUAL INTERNATIONAL CONFERENCE ON MICROELECTRO MECHANICAL SYSTEMS. MEMS 2002. LAS VEGAS, NV, JAN. 20 - 24, 2002, IEEE INTERNATIONAL MICRO ELECTRO MECHANICAL SYSTEMS CONFERENCE, NEW YORK, NY : IEEE, US, vol. CONF. 15, 2002, pages 133-136, XP010577613 ISBN: 0-7803-7185-2

## Description

### BACKGROUND OF THE INVENTION

### 1. Field of the Invention

The present invention relates to a preparation chip system for extracting DNA from a sample solution as a biological material for conducting a gene test, and to a method for DNA extraction from biological materials using the preparation chip system.

### 2. Description of the Related Art

To test a living body with a DNA sequencer, a DNA chip, and the like at a gene level, it is necessary to extract DNA from a biological material. A method is known to separate a desired analyte from a fluid sample, using a chamber, a storage chamber, and a cartridge obtained by assembling a detecting process region or the like on a fine fluid chip and leading the flow, particularly the diversion of a sample solution, a reagent, a waste solution and the like, by using a diverter including a capillary or a hydrophobic film. Such a method is described in, for example, the Japanese patent application JP 2001-527220.

In the conventional technique, to divert a waste solution, a diverter for passing a solution simply in the case where a limit back pressure is generated is used. In the case of applying the conventional technique to the case where the number of branches is large such as the case of sequentially passing a plurality of reagents, a plurality of limit back pressures have to be set, and it is difficult to reliably pass the reagents.

### SUMMARY OF THE INVENTION

A problem underlying the present invention is to provide a biological material preparation chip system with a simpler configuration, realizing a reliable flow of solutions, with improved reliability, and realizing prompt processing also in the case where a plurality of reagents have to be fed like in a preparation of extracting DNA from a sample solution.

Further, a method for extracting DNA from biological materials using improved preparation chip systems is to be provided.

The above problems are solved according to the independent claims. The dependent claims relate to preferred embodiments of the concept of the present invention.

To solve the problem of the conventional technique, the present invention provides a preparation chip system, provided for extracting DNA from a biological material, for injecting a sample solution into a preparation chip, mixing the sample solution with a dissolving solution to expose DNA, passing the dissolved sample solution through a carrier part to adsorb the DNA on the surface of a carrier, passing a cleaning solution through the carrier part to wash away the sample solution remaining on the surface, passing an eluting solution through the carrier part to elute the adsorbed DNA, and taking out the eluting solution containing the DNA, the system comprising:
a sample chamber in which the sample is injected,
- a dissolving solution chamber containing the dissolving solution,
- a first cleaning solution chamber containing a cleaning solution,
- a second cleaning solution chamber containing a cleaning solution,
- an eluting solution chamber containing the eluting solution,
- a mixing passage connected to the sample chamber and the dissolving solution chamber for mixing the sample and the dissolving solution with each other,
- a carrier part connected to the mixing passage,
- a waste chamber connected to the carrier part via a holding passage for temporarily holding a sample,
- a collection chamber connected to the waste chamber and holding the eluting solution passed through the carrier part,
- a first resistance part disposed in a first passage connecting the first cleaning solution chamber with both the mixing passage and the carrier part,
- a second resistance part disposed in a second passage connecting the second cleaning solution chamber with the carrier part side of the first passage,
- a third resistance part disposed between the eluting solution chamber and both of the mixing passage and the carrier part,
   and
- switchable ports for supplying solutions or air and switchable ports for discharging solutions or supplying air,
   wherein
- the first resistance part, the second resistance part and the third resistance part comprise a plurality of resistive materials,
- the carrier provided in the carrier part has a surface made of a glass material,
- the resistive material provided in the first, second and third resistance parts is the same material as that of the carrier, and
- the number of resistive materials in the second passage is larger than that in the first passage,
   and
- the eluting solution is passed through the carrier part by pressure from a pressure source,
and wherein
the magnitude relations of the passage flow resistances between the mixing passage, the carrier part and the resistance parts are as follows:
passage 120 < carrier part 130 < resistance part 160
resistance part 160 < resistance part 161
resistance part 160 < resistance part 162
resistance part 160 + resistance part 161 < resistance part 163
resistance part 160 + resistance part 161 < resistance part 164.

According to preferred embodiments of the preparation chip system, the resistive material is any of glass beads, glass wool, glass sintered body, and porous glass.

According to the invention, the passage for passing the cleaning solution and the eluting solution is ramified while a passage resistor being set by using a plurality of resistive materials. Therefore, also in the case where a plurality of reagents have to be fed like in a preparation of extracting DNA from a sample solution, a simpler configuration, reliable flow of the solutions, and improved reliability can be realized.

The method of the present invention for extracting DNA from a biological material in a preparation chip by
- injecting a liquid sample containing a biological material into the preparation chip,
- mixing the sample solution with a dissolving solution to dissolve the biological material and to expose DNA in the preparation chip,
- passing the dissolved sample solution through a carrier part of the preparation chip to adsorb the DNA on the surface of a carrier,
- passing a cleaning solution through the carrier part to wash out the sample solution remaining on the carrier surface,
- passing an eluting solution through the carrier part to elute the DNA adsorbed on the carrier surface,
   and
- taking out the eluting solution containing the DNA
is **characterized in that** a preparation chip system or a preparation chip according to claim 1 or 2 is used.

### BRIEF DESCRIPTION OF THE DRAWINGS

FIG 1 is a top view of a preparation chip according to an embodiment of the invention;
FIG. 2 is a perspective view showing the details of a resistance part in FIG 1;
FIG 3 is a flowchart showing a preparation process according to an embodiment; and
FIG 4 is a perspective view showing an outline of a preparation chip apparatus according to an embodiment.

### DESCRIPTION OF THE PREFERRED EMBODIMENTS

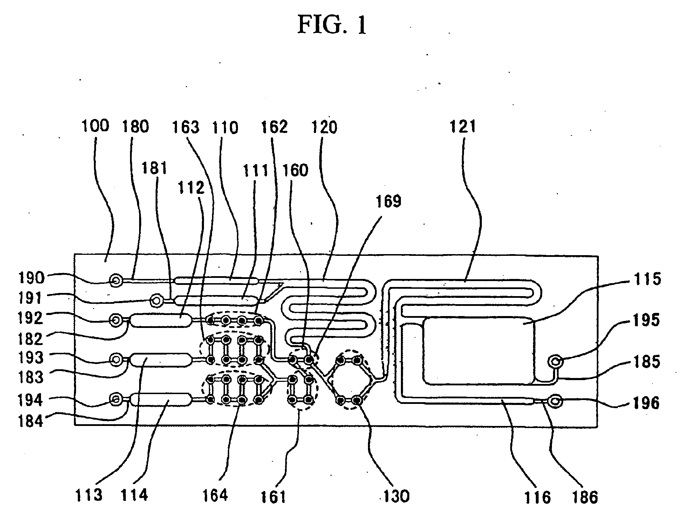

Referring to FIG 1, the structure of a preparation chip for extracting DNA from a sample solution as an embodiment will be described.

A preparation chip 100 has a sample chamber 110 in which a sample containing a biological material is injected, a dissolving solution chamber 111 containing a dissolving solution, cleaning solution chambers 112 and 113 containing cleaning solution, an eluting solution chamber 114 containing an eluting solution, a passage 120 for mixing the sample with the eluting solution, a carrier part 130 made of a plurality of carriers as substances for adsorbing DNA in the sample thereon and effectively performing chemical/physical operation of a small amount of an element and compound; a holding passage 121 for temporarily holding the sample, the dissolving solution, the cleaning solution, and the eluting solution passed through the carrier part 130, a waste chamber 115 for holding the sample, the dissolving solution, and the cleaning solution passed through the carrier part 130, and a collection chamber 116 for holding the eluting solution passed through the carrier part 130.

The chambers 110 to 116 are connected to ports 190 to 196 via port passages 180 to 186, respectively. Further, between the both of the mixing passage 120 and the carrier part 130 and the group consisting of the first and second cleaning solution chambers 112, 113 and the eluting solution chamber 114, resistance parts 160 to 164 made from a plurality of resistive materials 169 are provided, with connection passages being interposed among them.

The resistance part 162 is disposed in a first passage connecting the first cleaning solution chamber 112 with both the mixing passage 120 and the carrier part 130. The resistance part 163 is disposed in a second passage connecting the carrier part side of the first passage and the second cleaning solution chamber 113.

The resistance part 164 is disposed between the eluting solution chamber 114 and the carrier part 130, the resistance part 161 is disposed between the resistance parts 163, 164 and the carrier part 130, and the resistance part 160 is disposed between the resistance parts 161, 162 and the both of the passage 120 and the carrier part 130. A suitable carrier has the surface made of a glass material such as glass beads, glass wool, glass sintered body, porous glass, since such material efficiently adsorbs DNA.

The magnitude relations of the passage resistances between the passage 120, the carrier part 130, and the resistance parts 160 to 164 are as follows.
Passage 120 < carrier part 130 < resistance part 160
Resistance part 160 < resistance part 161
Resistance part 160 < resistance part 162
Resistance part 160 + resistance part 161 < resistance part 163
Resistance part 160 + resistance part 161 < resistance part 164

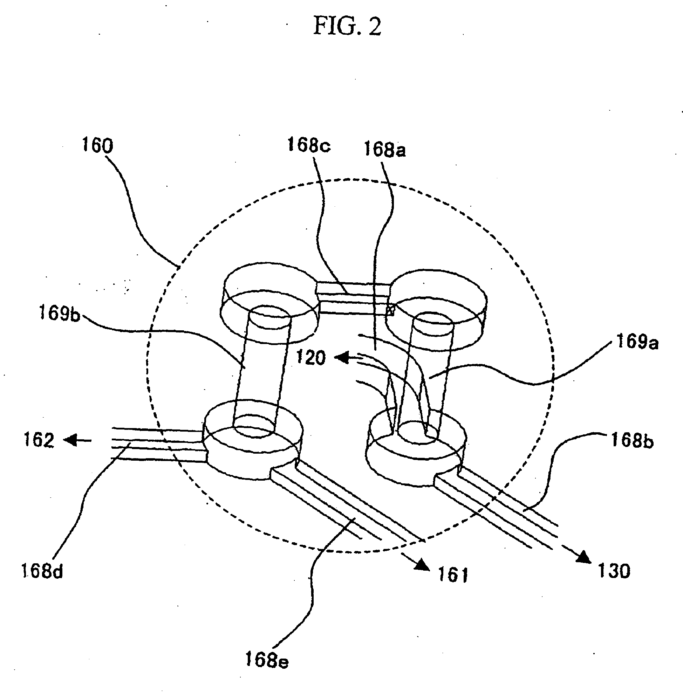

FIG. 2 is a perspective view showing the details of the resistance part 160. In the resistance part 160, two resistive materials 169a and 169b are connected in series via a connection passage 168c. Connection passages 168a and 168b are connected to the resistive material 169a, and connection passages 168d and 168e are connected to the resistive material 169b. The passage 120 is positioned at the end of the connection passage 168a. The carrier part 130 is positioned at the end of the connection passage 168b. The resistance part 162 is positioned at the end of the connection passage 168d. The resistance part 161 is positioned at the end of the connection passage 168e.

As the resistive material, it is preferable to use the same material as that of the carrier. Accordingly, to set the passage resistance to a proper value, it is sufficient to set the number of carriers. As compared with the case of setting passage resistance and the like by a single resistive material, not only the value itself but variations of the quality and the like are smaller. Thus, it is easier to assure reliability.

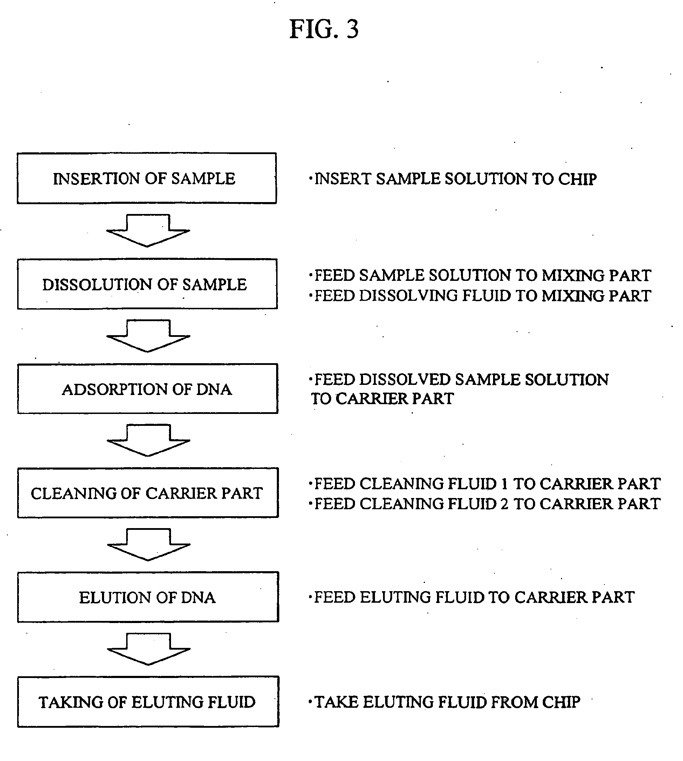

Outline of the preparing procedure will be described with reference to FIG. 3. The preparation corresponds to the range of up to extracting DNA from a biological material and consists of four processes of (1) dissolution, (2) adsorption, (3) cleaning, and (4) elution.

First, a sample solution is injected into the chip and mixed with a dissolving solution in the chip to dissolve the biological material and expose DNA (dissolving process). Next, the dissolved sample solution is passed through the carrier part where the DNA is adsorbed on the surface of the carrier (adsorbing process).

A cleaning solution is passed through the carrier part to wash away the sample solution remaining on the surface of the carrier (cleaning process). An eluting solution is passed through the carrier part to elute the DNA adsorbed on the surface of the carrier (eluting process). Finally, the eluting solution containing the DNA is taken out.

The procedure of performing the above-described preparing process in the chip will be described.

In the initial state, reagents are contained in the chip. Specifically, the dissolving solution chamber 111 is filled with the dissolving solution, the cleaning solution chamber 112 is filled with a cleaning solution, the cleaning solution chamber 113 is filled with another cleaning solution, and the eluting solution chamber 114 is filled with the eluting solution.

A sample solution is injected from the port 190 into the chip in the initial state to fill the sample chamber 110. At this time, the ports 191, 192, 193, and 194 are closed. At least one of the ports 195 and 196 is open to inject the sample solution from the port 190.

The sample solution in the sample chamber 110 and the dissolving solution in the dissolving solution chamber 111 are fed to the passage 120 where they are mixed. In this instance, the ports 192, 193, and 194 are closed, at least one of the ports 195 and 196 is opened, and air is injected from the ports 190 and 191. As necessary, the mixture of the sample and the dissolving solution may be heated in the chip.

The mixture of the sample solution and the eluting solution is fed from the passage 120 via the carrier part 130 to the passage 121. In this instance, the ports 192, 193, and 194 are closed, at least one of the port 195 and 196 is opened, and air is injected from the port 190 or 191. Since the passage resistance in the carrier part 130 is lower than that in the resistance part 160, the mixture from the passage 120 flows to the carrier part 130.

In the case of passing the mixture in the passage 121 via the carrier part 130 to the passage 120, the ports 192, 193, and 194 are closed, at least one of the ports 190 and 191 is opened, and air is injected from the port 195 or 196. Since the passage resistance in the passage 120 is lower than that in the resistance part 160, the mixture from the carrier part 130 flows to the passage 120. When the mixture passes through the carrier part 130, DNA in the mixture is adsorbed on the surface of the carrier. In the case of passing the mixture in the passage 121 to the waste chamber 115, the ports 192, 193, 194, and 196 are closed, the port 195 is opened, and air is injected from the port 190 or 191. In such a manner, the mixture is held in the waste chamber 115.

The cleaning solution is once fed from the cleaning solution chamber 112 to the passage 120. The ports 193, 194, 195, and 196 are closed, at least one of the ports 190 and 191 is opened, and air is injected from the port 192. Since the passage resistance in the resistance part 160 is lower than that in the resistance part 161, the cleaning solution from the cleaning solution chamber 112 flows to the resistance part 160. Since the passage resistance in the passage 120 is lower than that in the carrier part 130, the cleaning solution from the resistance part 160 flows to the passage 120. The cleaning solution is temporarily held in the passage 120.

The cleaning solution is fed from the passage 120 via the carrier part 130 to the passage 121. The ports 192, 193, and 194 are closed, at least one of the ports 195 and 196 is opened, and air is injected from the port 190 or 191. Since the number of carriers in the carrier part 130 is smaller than that in the resistance part 160 and the passage resistance is lower, the cleaning solution from the passage 120 flows to the carrier part 130. In the case of passing the cleaning solution in the passage 121 via the carrier part 130 to the passage 120, the ports 192, 193, and 194 are closed, at least one of the ports 190 and 191 is opened, and air is injected from the port 195 or 196. Since passage resistance in the passage 120 is lower than that in the resistance part 160, the cleaning solution from the carrier part 130 flows to the passage 120.

When the cleaning solution passes through the carrier part 130, components other than the DNA on the carrier surface are washed out. In the case of passing the cleaning solution in the passage 121 to the waste chamber 115, the ports 192, 193, 194 and 196 are closed, the port 195 is opened, and air is injected from the port 190 or 191. In such a manner, following the mixture, the cleaning solution is held in the waste chamber 115.

The cleaning solution is once fed from the cleaning solution chamber 113 to the passage 120. The ports 192, 194, 195, and 196 are closed, at least one of the ports 190 and 191 is opened, and air is injected from the port 193. Since the passage resistance in the resistance part 161 is lower than that in the resistance part 164, the cleaning solution from the cleaning solution chamber 113 flows to the resistance part 161.

Since the passage resistance in the resistance part 160 is lower than that in the resistance part 162, the cleaning solution from the resistance part 161 flows to the resistance part 160. Since the passage resistance in the passage 120 is lower than that in the carrier part 130, the cleaning solution from the resistance part 160 flows to the passage 120. The cleaning solution is temporarily held in the passage 120.

The cleaning solution is fed from the passage 120 via the carrier part 130 to the passage 121. The ports 192, 193, and 194 are closed, at least one of the ports 195 and 196 is opened, and air is injected from the port 190 or 191. Since the passage resistance in the carrier part 130 is lower than that in the resistance part 160, the cleaning solution from the passage 120 flows to the carrier part 130.

In the case of passing the cleaning solution in the passage 121 via the carrier part 130 to the passage 120, the ports 192, 193, and 194 are closed, at least one of the ports 190 and 191 is opened, and air is injected from the port 195 or 196. Since passage resistance in the passage 120 is lower than that in the resistance part 160, the cleaning solution from the carrier part 130 flows to the passage 120. When the cleaning solution passes through the carrier part 130, the components other than DNA on the carrier surface are further washed.

In the case of passing the cleaning solution in the passage 121 to the waste chamber 115, the ports 192, 193, 194, and 196 are closed, the port 195 is opened, and air is injected from the port 190 or 191. Following the mixture and the cleaning solution, the cleaning solution is held in the waste chamber 115.

The eluting solution is once fed from the eluting solution chamber 114 to the passage 120. The ports 192, 193, 195, and 196 are closed, at least one of the ports 190 and 191 is opened, and air is injected from the port 194. Since the passage resistance in the resistance part 161 is lower than that in the resistance part 163, the eluting solution from the eluting solution chamber 114 flows to the resistance part 161. Since the passage resistance in the resistance part 160 is lower than that in the resistance part 162, the eluting solution from the resistance part 161 flows to the resistance part 160.

Further, since the passage resistance in the passage 120 is lower than that in the carrier part 130, the eluting solution from the resistance part 160 flows to the passage 120. The eluting solution is temporarily held in the passage 120.

The eluting solution is fed from the passage 120 via the carrier part 130 to the passage 121. The ports 192, 193, and 194 are closed, at least one of the ports 195 and 196 is opened, and air is injected from the port 190 or 191. Since the passage resistance in the carrier part 130 is lower than that in the resistance part 160, the eluting solution from the passage 120 flows to the carrier part 130. In the case of passing the eluting solution in the passage 121 via the carrier part 130 to the passage 120, the ports 192, 193, and 194 are closed, at least one of the ports 190 and 191 is opened, and air is injected from the port 195 or 196. Since passage resistance in the passage 120 is lower than that in the resistance part 160, the eluting solution from the carrier part 130 flows to the passage 120. When the eluting solution passes through the carrier part 130, the DNA is eluted from the carrier surface and is retained in the eluting solution.

In the case of passing the eluting solution in the passage 121 to the collection chamber 116, the ports 192, 193, 194 and 195 are closed, the port 196 is opened, and air is injected from the port 190 or 191. In such a manner, the eluting solution retaining the DNA is held in the collection chamber 116.

Finally, the eluting solution held in the collection chamber 116 is taken out from the port 196, and the preparation is finished. The eluting solution retaining the DNA after the preparation is amplified as necessary and used for a test of a living body at a gene level using a DNA sequencer, a DNA chip, and the like.

As described above, the resistance parts 160 to 164 are disposed between the both of the passage 120 and the carrier part 130 and the group consisting of the cleaning solution chambers 112, 113 and the eluting solution chamber 114, and the magnitude relations of the passage resistances between the passage 120, the carrier part 130, and the resistance parts 160 to 164 can be set according to the number of carriers. Therefore, it is easy to dispose the components in such a state that setting of the passage resistance is flexible.

Also in the case of changing the passage resistance in accordance with fluid properties, the passage resistance can be set by adjusting the number of resistive materials. Further, by changing the arrangement of the resistive materials, the flow direction control can be changed without forming a new chip.

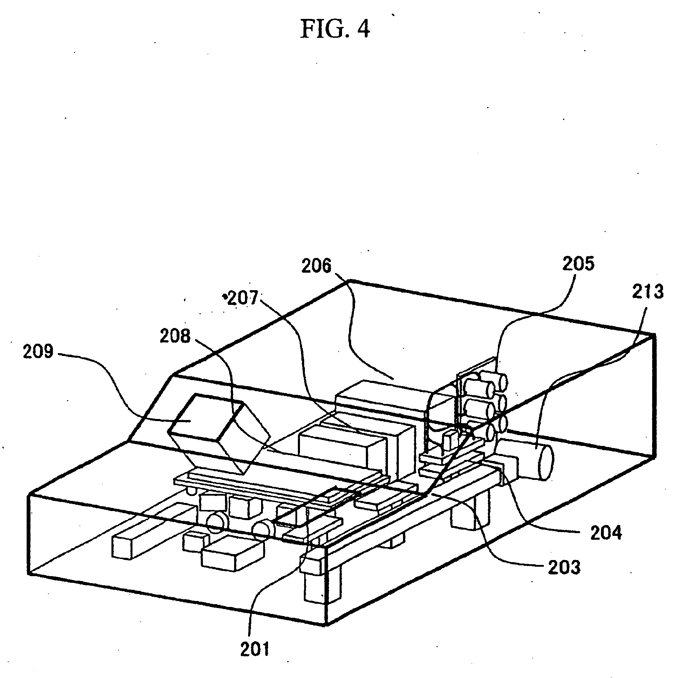

A preparation chip system (apparatus) of FIG. 4 has a chip receiving window 201 to which a preparation chip is inserted, a movement stage 203 for moving the preparation chip, a preparation stage 204 for performing the preparation, a valve 205 and a pump 213 for passing solutions in the preparation chip, a power supply 206, a motor driver 207, a control board 208, and an information access panel 209. The motor driver 207 and the control board 208 are used for operating the movement stage 203, the valve 205, and the pump 213. The power supply 206 supplies electricity to various parts. The information access panel 209 is used for inputting measurement parameters and outputting a measurement result.

The preparation chip apparatus can extract DNA from a biological material in order to conduct a gene test. A preparation chip is inserted from the chip receiving window 201.

In the preparation chip, reagents are filled, and a sample containing a biological material is injected. The preparation chip is carried by the movement stage 203 to the preparation stage 204. In the preparation stage 204, the sample solution containing the biological material is mixed with the dissolving solution to dissolve the biological material and expose DNA, in the preparation chip.

The dissolved sample solution is fed through the carrier part to adsorb the DNA on the carrier surface. The cleaning solution is passed through the carrier part to wash away the sample solution remaining on the carrier surface. The eluting solution is passed through the carrier part to elute the DNA adsorbed on the carrier surface.

The preparation process is performed automatically in the preparation chip apparatus, and the preparation chip is carried by the movement stage 203 to the chip receiving window 201 and is taken out. It is sufficient to load the preparation chip in the preparation chip apparatus and it is unnecessary to accurately control the amount of a solution passed.

## Claims

1. Preparation chip system, provided for extracting DNA from a biological material, for injecting a sample solution into a preparation chip (100), mixing the sample solution with a dissolving solution to expose DNA, passing the dissolved sample solution through a carrier part (130) to adsorb the DNA on the surface of a carrier, passing a cleaning solution through the carrier part (130) to wash out the sample solution remaining on the surface, passing an eluting solution through the carrier part (130) to elute the adsorbed DNA, and taking out the eluting solution containing the DNA, the system comprising:
- a sample chamber (110) in which the sample is injected,
- a dissolving solution chamber (111) containing the dissolving solution,
- a first cleaning solution chamber (112) containing a cleaning solution,
- a second cleaning solution chamber (113) containing a cleaning solution,
- an eluting solution chamber (114) containing the eluting solution,
- a mixing passage (120) connected to the sample chamber (110) and the dissolving solution chamber (111) for mixing the sample and the dissolving solution with each other,
- a carrier part (130) connected to the mixing passage (120),
- a waste chamber (115) connected to the carrier part via a holding passage (121) for temporarily holding a sample,
- a collection chamber (116) connected to the waste chamber (115) and holding the eluting solution passed through the carrier part (130),
- a first resistance part (162) disposed in a first passage connecting the first cleaning solution chamber (112) with both the mixing passage (120) and the carrier part (130),
- a second resistance part (163) disposed in a second passage connecting the second cleaning solution chamber (113) with the carrier part side of the first passage,
- a third resistance part (164) disposed between the eluting solution chamber (114) and both of the mixing passage (120) and the carrier part (130),
and
- switchable ports (190, 191, 192, 193, 194) for supplying solutions or air and switchable ports (195, 196) for discharging solutions or supplying air,
wherein
- the first resistance part (162), the second resistance part (163) and the third resistance part (164) comprise a plurality of resistive materials (169),
- the carrier provided in the carrier part (130) has a surface made of a glass material,
- the resistive material (169) provided in the first, second and third resistance parts (162, 163, 164) is the same material as that of the carrier, and
- the number of resistive materials in the second passage is larger than that in the first passage,
and
- the eluting solution is passed through the carrier part (130) by pressure from a pressure source,
and wherein
the magnitude relations of the passage flow resistances between the mixing passage (120), the carrier part (130) and the resistance parts (160 to 164) are as follows:
passage 120 < carrier part 130 < resistance part 160
resistance part 160 < resistance part 161
resistance part 160 < resistance part 162
resistance part 160 + resistance part 161 < resistance part 163
resistance part 160 + resistance part 161 < resistance part 164.

2. Preparation chip system according to claim 1, wherein the resistive material is any of glass beads, glass wool, glass sintered body, and porous glass.

3. Method for extracting DNA from a biological material in a preparation chip by
- injecting a liquid sample containing a biological material into the preparation chip,
- mixing the sample solution with a dissolving solution to dissolve the biological material and to expose DNA in the preparation chip,
- passing the dissolved sample solution through a carrier part of the preparation chip to adsorb the DNA on the surface of a carrier,
- passing a cleaning solution through the carrier part to wash out the sample solution remaining on the carrier surface,
- passing an eluting solution through the carrier part to elute the DNA adsorbed on the carrier surface,
and
- taking out the eluting solution containing the DNA,
**characterized in that** a preparation chip system according to claim 1 or 2 is used.

## Patentansprüche

1. Präparatives Chipsystem, das vorgesehen ist zur Gewinnung von DNA aus einem biologischen Material durch Einspritzen einer Probenlösung in einen präparativen Chip (100), Mischen der Probenlösung mit einer Auflösungslösung zur Freisetzung der DNA, Durchleiten der gelösten Probenlösung durch einen Trägerabschnitt (130) zur Adsorption der DNA an der Oberfläche eines Trägers, Durchleiten einer Reinigungslösung durch den Trägerabschnitt (130) zum Auswaschen der auf der Oberfläche verbliebenen Probenlösung, Durchleiten einer Elutionslösung durch den Trägerabschnitt (130) zur Elution der adsorbierten DNA und Entnahme der die DNA enthaltenden Elutionslösung,
wobei das System aufweist:
- eine Probenkammer (110), in welche die Probe eingespritzt wird,
- eine Auflösungslösungskammer (111), welche die Auflösungslösung enthält,
- eine erste Reinigungslösungskammer (112), die eine Reinigungslösung enthält,
- eine zweite Reinigungslösungskammer (113), die eine Reinigungslösung enthält,
- eine Elutionslösungskammer (114), welche die Elutionslösung enthält,
- eine Mischpassage (120), die mit der Probenkammer (110) und der Auflösungslösungskammer (111) verbunden ist, zum Mischen der Probe und der Auflösungslösung miteinander,
- einen Trägerabschnitt (130), der mit der Mischpassage (120) verbunden ist,
- eine Abfallkammer (115), die über eine Haltepassage (121) zur vorübergehenden Aufnahme einer Probe mit dem Trägerabschnitt verbunden ist,
- eine Sammelkammer (116), die mit der Abfallkammer (115) verbunden ist und die durch den Trägerabschnitt (130) hindurchgegangene Elutionslösung aufnimmt,
- einen ersten Widerstandsbereich (162), der in einem ersten Durchgang vorgesehen ist, der die erste Reinigungslösungskammer (112) mit der Mischpassage (120) sowie mit dem Trägerbereich (130) verbindet,
- einen zweiten Widerstandsbereich (163), der in einem zweiten Durchgang vorgesehen ist, der die zweite Reinigungslösungskammer (113) mit der Trägerbereichsseite des ersten Durchgangs verbindet,
- einen dritten Widerstandsbereich (164), der zwischen der Elutionslösungskammer (114) und der Mischpassage (120) sowie dem Trägerabschnitt (130) vorgesehen ist,
und
- schaltbare Einlässe (190, 191, 192, 193, 194) zum Einführen von Lösungen oder von Luft sowie schaltbare Auslässe (195, 196) zur Abgabe von Lösungen oder eingeführter Luft,
wobei
- der erste Widerstandsbereich (162), der zweite Widerstandsbereich (163) und der dritte Widerstandsbereich (164) eine Vielzahl von Strömungswiderstandsmaterialien (169) aufweisen,
- der im Trägerbereich (130) vorgesehene Träger eine aus einem Glasmaterial bestehende Oberfläche besitzt,
- das im ersten, zweiten und dritten Widerstandsbereich (162, 163, 164) vorgesehene Strömungswiderstandsmaterial (169) das gleiche Material ist wie das des Trägers
und
- die Anzahl der Strömungswiderstandsmaterialien im zweiten Durchgang größer ist als die im ersten Durchgang
und
- die Elutionslösung durch Druck von einer Druckquelle durch den Trägerbereich (130) hindurchgeleitet wird,
und wobei
die Größenbeziehungen der Durchgangs-Strömungswiderstände zwischen der Mischpassage (120), dem Trägerbereich (130) und
den Widerstandsbereichen (160 bis 164) wie folgt sind:
Passage 120 < Trägerbereich 130 < Widerstandsbereich 160
Widerstandsbereich 160 < Widerstandsbereich 161
Widerstandsbereich 160 < Widerstandsbereich 162
Widerstandsbereich 160 + Widerstandsbereich 161 < Widerstandsbereich 163
Widerstandsbereich 160 + Widerstandsbereich 161 < Widerstandsbereich 164.

2. Präparatives Chipsystem nach Anspruch 1, bei dem das Strömungswiderstandsmaterial aus Glaskügelchen, Glaswolle, einem Glas-Sinterkörper oder aus porösem Glas besteht.

3. Verfahren zur Gewinnung von DNA aus einem biologischen Material in einem präparativen Chip durch
- Einspritzen einer flüssigen Probe, die ein biologisches Material enthält, in den präparativen Chip,
- Mischen der Probenlösung mit einer Auflösungslösung zum Lösen des biologischen Materials und zur Freisetzung der DNA im präparativen Chip,
- Durchleiten der gelösten Probenlösung durch einen Trägerabschnitt des präparativen Chips zur Adsorption der DNA an der Oberfläche eines Trägers,
- Durchleiten einer Reinigungslösung durch den Trägerabschnitt zum Auswaschen der auf der Trägeroberfläche verbliebenen Probenlösung,
- Durchleiten einer Elutionslösung durch den Trägerabschnitt zur Elution der auf der Trägeroberfläche adsorbierten DNA
und
- Entnahme der Elutionslösung, welche die DNA enthält,
**dadurch gekennzeichnet, dass** ein präparatives Chipsystem nach Anspruch 1 oder 2 verwendet wird.

## Revendications

1. Système de puce de préparation, prévu pour extraire de l'ADN d'un matériau biologique, pour injecter une solution d'échantillon dans une puce de préparation (100), pour mélanger la solution d'échantillon avec une solution de dissolution afin d'exposer l'ADN, pour faire passer la solution d'échantillon dissoute à travers une partie de support (130) afin d'adsorber l'ADN à la surface d'un support, pour faire passer une solution de nettoyage à travers la partie de support (130) afin de nettoyer la solution d'échantillon restant à la surface, pour faire passer une solution d'élution à travers la partie de support (130) afin d'éluer l'ADN adsorbé et pour extraire la solution d'élution contenant l'ADN, le système comprenant :
- une chambre d'échantillon (110) dans laquelle l'échantillon est injecté,
- une chambre de solution de dissolution (111) contenant la solution de dissolution,
- une première chambre de solution de nettoyage (112) contenant une solution de nettoyage,
- une seconde chambre de solution de nettoyage (113) contenant une solution de nettoyage,
- une chambre de solution d'élution (114) contenant la solution d'élution,
- un passage de mélange (120) relié à la chambre d'échantillon (110) et à la chambre de solution de dissolution (111) pour mélanger ensemble l'échantillon et la solution de dissolution,
- une partie de support (130) reliée au passage de mélange (120),
- une chambre à déchets (115) reliée à la partie de support via un passage de stockage (121) permettant de stocker temporairement un échantillon,
- une chambre de collecte (116) reliée à la chambre à déchets (115) et contenant la solution d'élution passant à travers la partie de support (130),
- une première partie de résistance (162) disposée dans un premier passage reliant la première chambre de solution de nettoyage (112) à la fois au passage de mélange (120) et à la partie de support (130),
- une seconde partie de résistance (163) disposée dans un second passage reliant la seconde chambre de solution de nettoyage (113) au premier passage, du côté de la partie de support,
- une troisième partie de résistance (164) disposée entre la chambre de solution d'élution (114) d'un côté et le passage de mélange (120) et la partie de support (130) de l'autre,
et
- des orifices commutables (190, 191, 192, 193, 194) prévus pour fournir des solutions ou de l'air et des orifices commutables (195, 196) prévus pour évacuer les solutions ou fournir de l'air,
dans lequel
- la première partie de résistance (162), la seconde partie de résistance (163) et la troisième partie de résistance (164) comprennent une pluralité de matériaux résistifs (169),
- le support disposé dans la partie de support (130) possède une surface composée d'un matériau de verre,
- le matériau résistif (169) disposé dans les première, seconde et troisième parties de résistance (162, 163, 164) est le même matériau que celui du support, et
- le nombre de matériaux résistifs dans le second passage est plus grand que dans le premier passage,
et
- la solution d'élution passe à travers la partie de support (130) sous l'effet de la pression provenant d'une source de pression,
et dans lequel
les rapports de grandeur des résistances à l'écoulement entre le passage de mélange (120), la partie de support (130) et les parties de résistance (160 à 164) sont les suivants :
passage 120 < partie de support 130 < partie de résistance 160
partie de résistance 160 < partie de résistance 161
partie de résistance 160 < partie de résistance 162
partie de résistance 160 + partie de résistance 161 < partie de résistance 163
partie de résistance 160 + partie de résistance 161 < partie de résistance 164.

2. Système de puce de préparation selon la revendication 1, dans lequel le matériau résistif est composé indifféremment de perles de verre, de fibres de verre, de verre fritté ou de verre poreux.

3. Méthode d'extraction de l'ADN d'un matériel biologique dans une puce de préparation en
- injectant un échantillon liquide contenant un matériel biologique dans la puce de préparation,
- mélangeant la solution d'échantillon avec une solution de dissolution pour dissoudre le matériel biologique et exposer l'ADN dans la puce de préparation,
- faisant passer la solution d'échantillon dissoute à travers une partie de support de la puce de préparation pour adsorber l'ADN à la surface d'un support,
- faisant passer une solution de nettoyage à travers la partie de support pour nettoyer la solution d'échantillon restant à la surface du support,
- faisant passer une solution d'élution à travers la partie de support pour éluer l'ADN adsorbé à la surface du support,
et
- extrayant la solution d'élution contenant l'ADN,
**caractérisé en ce qu'**un système de puce de préparation selon la revendication 1 ou 2 est utilisé.
